# EUROPEAN PATENT APPLICATION

(11) **EP 2 182 055 A1**
(43) Date of publication of application: **05.05.2010**
(21) Application number: 08166605.9
(22) Date of filing: 14.10.2008
(51) Int. Cl.: C12N 5/0775

(54) **Human cord blood derived unrestricted somatic stem cells (USSC)**

(71) Applicant: Heinrich-Heine-Universität Düsseldorf, 40225 Düsseldorf (DE)
(72) Inventor: Kögler, Gesine, 40267 Düsseldorf (DE); Kluth, Simone Maria, 40625 Düsseldorf (DE)
(74) Representative: Krauss, Jan

(57) **Abstract**

Unrestricted somatic stem cell (USSC) derived from human umbilical cord blood, placental blood and/or blood samples from new-borns, said somatic stem cell being distinct from but capable to differentiate into mesenchymal cells (bone cells, chondrocytes), neural cells, endodermal cells, but not into haematopoietic lineage stem or progenitor cells and endothelial stem or progenitor cells and adipocytes **characterized in that** the USSC expresses the DLK1-gene the expression being detectable by a RT-PCR assay and/or on the protein level and that it is a clonally selected and expanded cell.

## Description

### Field of the invention

The present invention pertains to a somatic stem cell, a medicament comprising the stem cell of the invention and a method for purification, isolation and unique differentiation-potential of the stem cell of the invention.

### Background of the invention

Although stem cells are permanently self-replacing, they are generally slow cycling. Conventionally, it is thought that these cells give rise to a transient amplifying cell population with limited self-renewal capacity to increase numbers of differentiated cells. Hitherto, the challenge has been to locate stem cells in the adult human and, therefore, a number of surrogate markers (e. g. colony forming tests for the hematopoietic lineage) have been employed in the existing literature.

A number of US-Patents e. g. US 5,486,359; 5,591,625; 5,736,396; 5,811,094; 5,827,740; 5,837,539; 5,908,782; 5,908,784; 5,942,225; 5,965,436; 6,010,696; 6,022,540; 6,087,113; 5,858,390; 5,804,446; 5,846,796; 5,654,186; 6,054,121; 5,827,735; 5,906,934 are dealing with mesenchymal stem cells (MSC), which can be differentiated into several progenitor cells, for example muscle progenitor cells, connective tissue cell progenitors or oval cells. Muscle progenitor cells are differentiating further into cardiac, skeletal as well as smooth muscle cells, whereas the connective tissue cell progenitor may differentiate into bone, cartilage as well as fat. Oval cells can differentiate into liver or pancreas cells (Grompe etal, 2001).
The presence of non-hematopoietic stem cells within umbilical cord blood is still under discussion (Mayani et al. 2000, Mareschi et al. 2001). The German patent application DE 198 03 267 A1 was the first to describe osteoblast progenitors and bone formation from human cord blood.
However, the use of these mesenchymal progenitor cells of prior art is often limited since they are too far developed in order to be useful tools for generating organs or tissues. In other words, they seem to be already too much committed and specialized to yield a functional regenerating organ or tissue.

WO-A-02/036751 discloses a composition in human cord and placental blood which comprises unrestricted somatic stem cells is described here which can be amplified in vitro to large quantities sufficient for medical applications as regenerative medicines. An initiation and maintenance as well as ex vivo expansion protocol of such stem cells from cord blood is described. Furthermore, it is shown that from these cells employing varying differentiation induction protocols distinct lineage progenitors for haematopoiesis and endothelium, as well as mesenchymal progenitors for muscle bone, cartilage and fat as well as neural progenitors can be cultured and expanded for use in regenerative medicine.

### Summary of the invention

The present invention is based on the surprising result, that a stable cell line could be isolated from cord blood having multipotent potential and is different from USSC according to WO-A-02/036751.
The unrestricted somatic stem cell (USSC) of the invention can be derived from human umbilical cord blood, placental blood and/or blood samples from new-borns, said somatic stem cell being distinct from but capable to differentiate into mesenchymal cells (bone cells, or chondrocytes), neural cells, endoderm cells, Unlike the USSC according to WO-A-02/036751 the cell of the invention does not differentiate into haematopoietic lineage stem or progenitor cells and endothelial stem or progenitor cells. The cell can be isolated and cultured/expanded on a clonal level and discriminated from other stem cells of prior art due to its capability to express the DLK1-gene, which expression is detectable e.g. by a RT-PCR assay and/or on the protein level.
In particular, the stem cell of the invention is adherent and CD45-, CD14-, CD34 negative.
Furthermore, the stem cell of the invention is reacting negatively with the markers Oct4-, Nanog-, Sox 2, SSEA1, SSEA 3, and SSEA 4 and is negative for telomerase activity. Subject of the present invention are also medicaments comprising somatic stem cells of the invention.
A further subject of the present invention is a method of using unrestricted somatic stem cells according to the invention in gene therapy, organ regeneration and replacement, testing of pharmaceuticals, testing of biomaterials including orthopedic devices, in vitro and vivo support and growth of blood vessels and support of hematopoietic cells, therapy of vascular and cardiac or smooth muscle diseases, bone, hepatic, pancreatic or neural diseases.
Still another subject of the invention is a method for the isolation and purification of unrestricted neonatal stem cells according to the invention comprising the steps of density gradient isolation, selection of a single cell by the AVISO Cell selection system, culture of clonal adherent cells and subculture applying growth factors.
The somatic stem cell of the invention is derived from human umbilical cord blood, placenta blood and/or the blood from a newborn child, said somatic stem cell being distinct from but capable to differentiate into mesenchymal stem or progenitor cells, neural stem or liver progenitor cells. These cells represent the progenitor of the mesenchymal stem cells as well as neural stem cells and endodermal stem cells. This unique multifunctional capacity and the technology to expand these cord blood (CB) derived unrestricted somatic stem cells (USSC) from a single cell on a clonal level, either as such somatic stem cells or as committed cells under distinct differentiation protocols, allows precise characterization, standardization and utilization for the production and implementation of stem cell therapy in regenerative medicine.
According to the invention a medicament (regenerative therapeutic) is provided comprising the somatic stem cells of the invention as well as a plurality or mixtures of somatic stem cells according to the invention. The medicament may further contain carrier substances or auxiliary substances, which are medically and pharmacologically acceptable. The present invention is also related with a method of using USSC or a plurality or mixtures of stem cells of the invention in gene therapy, organ replacement, testing of pharmaceutical, in vitro and in vivo growth of blood vessels, therapy of vascular, bone, hepatic, pancreatic and neural diseases and support of hematopoiesis in vitro and in vivo

For example, the USSCs of the present invention may be applied locally at the site of need, e. g. with or without biomaterials.
Depending on the kind of disease local and/or systemic administration of the USSCs is suitable. The USSCs may be applied directly or together with pharmaceutical acceptable carriers or adjuvants. It may be advantageous to add further substances which promote curing of the diseases. For example, in orthopedic applications substances which improve bone regeneration may be co-applied with the USSCs.
Basically, the methods known for the application of MSCs can be applied in an analogous manner when applying USSCs. Furthermore, the application of stem cells is described for example in B. E. Strauer et al. M. "Intrakoronare, humane autologe Stammzelltransplantation zur Myokardregeneration nach Herzinfarkt", Dtsch med Wochenschr 2001; 126: 932-938; Quarto R., et al. "Repair of Large Bone Defects with the Use of Autologous Bone Marrow Stromal Cells", N Engl J Med 2001; 344: 385-386; Vacanti C. A., "Brief Report: Replacement of an Avulsed Phalanx with Tissue-Engineered Bone" N Engl J Med 2001; 344: 1511-1514, May 17,2001; Hentz V. R., "Tissue Engineering for Reconstruction of the Thumb", N Engl J Med 2001; 344: 1541548 ; Brittberg M., "Treatment of Deep Cartilage Defects in the Knee with Autologous Chondrocyte Transplantation", N Engl J Med 1994; 331: 889-895, Oct 6,1994; Freed C. R.," Transplantation of Embryonic Dopamine Neurons for Severe Parkinson's Disease", N Engl J Med 2001; 344: 710-719; Shin'oka T., "Transplantation of a Tissue-Engineered Pulmonary Artery", N Engl J Med 2001; 344: 532-533. Shapiro A. M. J., Islet Transplantation in Seven Patients with Type 1 Diabetes Mellitus Using a Glucocorticoid-Free Immunosuppressive Regimen N Engl J Med 2000; 343: 230-238. These references are incorporated by reference.

### Brief description of the drawings

- Figure 1:: Expression of the stem cell markers Oct4, Nanog and Sox2 and the transcription factors Klf4 and cMyc. )
- Figure2:: Immunohistochemical staining of Oct4 and Nanog.

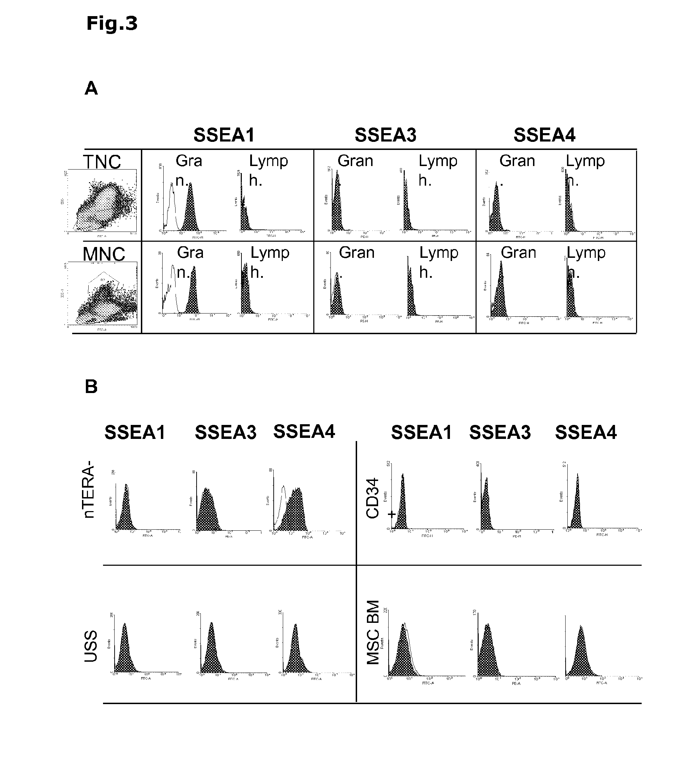

- Figure 3:: Flow cytometry expression analysis of SSEA1, SSEA3 and SSEA4.
- Figure 4:: Telomerase activity.
- Figure 5:: Expression of endodermal markers.
- Figure 6:: Functional analysis of endodermal differentiated USSCs.
- Figure 7 and 8:: Generation of single cell clones with a cell selector
- Figure 9:: RT-PCR on cell clones
- Figure 10:: RT-PCR - Dlk-1 expression in USSC cell-lines and clones, positive control
- Figure 11:: Oil red O staining of the lipid vesicles performed 2 wk after stimulation demonstrates ongoing adipogenisis

The stem cells of the invention are further described in greater detail.

### Detailed description of the invention

The present invention discloses that typical embryonic stem cell markers are not or are only weakly expressed in CB cells. USSC lines, CB mononuclear cells and CD34⁺ cells do not express OCT4, NANOG and SOX2 neither on transcript level (RT-PCR) nor on protein level (WB, IC), and furthermore do not show substantial or even any telomerase activity. In addition, it was possible to show that the markers SSEA1, SSEA3 and SSEA4 cannot be used in CB as markers of an embryonic-like phenotype. SSEA-1 recognizes the CD15 epitope, which is already expressed on granulocytes. SSEA4 also identifies an adult mesenchymal stem cell population¹¹ and SSEA3 was always tested negative¹².
Furthermore, it is disclosed that CB does not have to contain embryonic like cells, in fact it contains multipotent neonatal cells as USSC. Similar to fetal cells USSC have long telomeres and a high proliferative capacity. Besides their differentiation to mesodermal and ectodermal cells they are able to generate an endodermal precursor phenotype, but not all differentiation lineages, i.e., they are not pluripotent. This results as an example in a functional production of cytochrome Cyp3A4A, urea and human albumin, however to a much lower degree than its adult liver counterpart². For future analysis it will be important to compare the native neonatal cells from CB with the reprogrammed fetal fibroblast cells, reprogrammed CB -USSC and the true embryonic stem (ES) cells (physiological and malignant) in order to define the functional differentiation capacity of the cells also in relation to tumor formation.
The resulting expression pattern of all the ES-like markers and transcription factors described here show that neonatal cells from CB are not embryonic-like cells. Moreover, more than 10.000 allogeneic cord blood transplantations have been performed without any germ cell tumor (including teratoma).

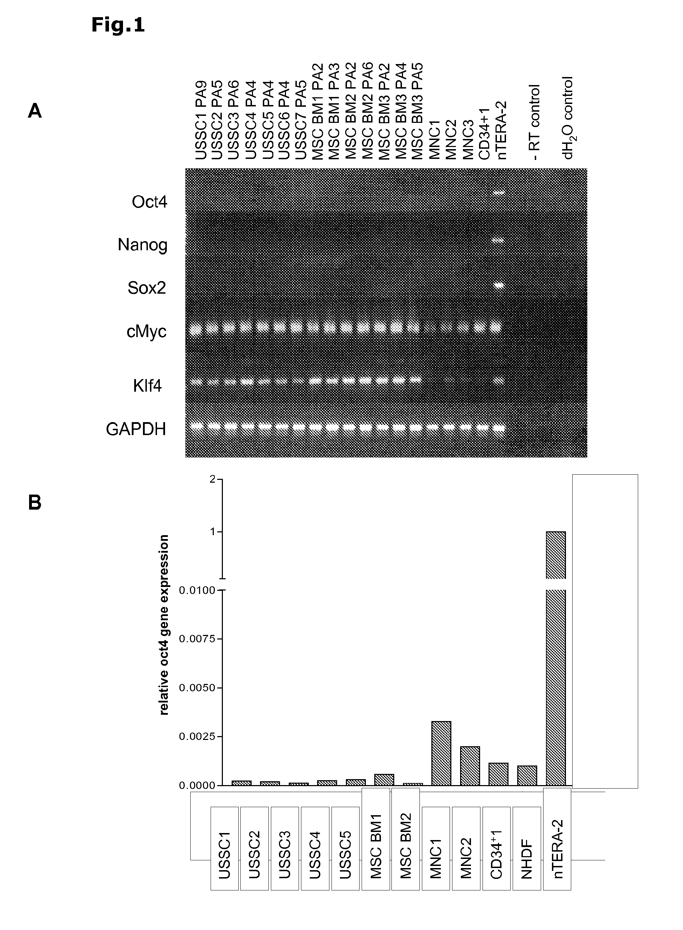

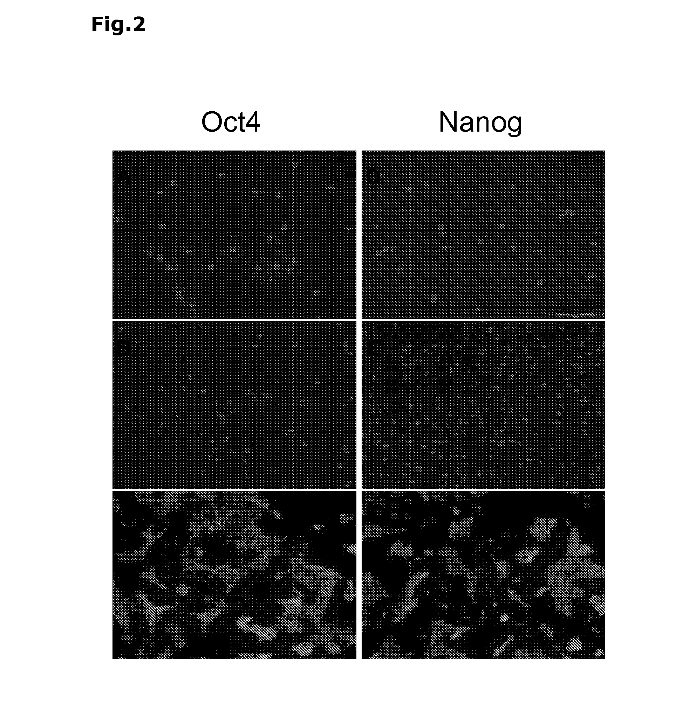

The potential of adult stem cells to differentiate into variant tissue cell types provides a valuable source for cellular therapeutics in transplantation. Cord blood is a promising and well established source of multipotent stem cells which exhibit the capacity to develop into tissues of all three germ layers *in vivo* and *in vitro*^{3,4}. This multipotent capacity of CB cells is in accordance with their non-tumorigenic potential. Only ES cells (especially of mouse origin), induced pluripotent stem cells (iPS) or naturally occurring delayed matured embryonic germ and stem cells harbour the risk of tumor formation.
Consequently, umbilical CB blood banking has become popular and CB derived stem cells are frequently used for allogeneic hematopoietic transplantation.
Compared to its allogeneic adult bone marrow counterpart CB has substantial logistic and clinical advantages. These include faster availability of banked CB units, the extension of the donor pool because of the tolerance of one or two in six HLA-mismatches, lower severity and incidence of acute graft versus host disease, lower risk of transmission of infections by latent viruses, and the absence of risks to donors.
The invention discloses a pluripotent adherent cell in cord blood. These cells have the potential to differentiate into mesodermal osteoblasts, chondroblasts, ectodermal cells of all three neural lineages as well as endodermal hepatic cells^{1,2,13-15} . However, no extra-embryonic lineages, including the trophoblast have been identified, indicating their restricted differentiation potency. The invention discloses the possible pluripotency characteristics of USSC as well as other cell populations in cord blood and bone marrow. The concept that ES-cells or ES-like cells circulate in the neonate and the CB itself is at present speculative.
The pluripotency markers OCT4, NANOG and the transcription factor SOX2 build a transcriptional network that regulates a large number of genes involved in cellular differentiation in ES cells. In contrast, SOX2 can be expressed without OCT3/4 and NANOG in various cell types, without generating pluripotency. Analyses of human ES cells revealed that all three factors occupy and activate genes that promote ES cell growth and self renewal while simultaneously repressing genes that promote differentiation. Downregulation of OCT3/4 and NANOG results in differentiation in physiological and malignant ES cells. *In vivo,* OCT4 is expressed within the inner cell mass of blastocysts from which ES cells are derived. OCT4 expression is down regulated in somatic cells around the time of gastrulation, but retained in primordial germ cells only and presumably some adult stem cell populations. However, no effect of knock out of Oct3/4 in adult stem cells of many different tissues has been identified, questioning its role in adult stem cells.
Nonetheless, some publications reported the existence of embryonic-like cells in CB blood^{5-9,16} In consideration of the analysis of the pluripotency status, especially based on the stem cell markers NANOG and OCT4, the results remain very questionable. Both harbour potential pitfalls for data misinterpretation due to pseudogenes and alternatively spliced variants¹⁷⁻²¹. The use of primer pairs which have the potential to bind pseudogenes and alternatively spliced variants results in amplification of false positive artefacts and undermines the hypothesis that cells are actually embryonic-like. The same problem can occur on protein level when OCT4 antibodies are used which cannot discriminate between the isoform 1 which is localized in the nucleus and isoform 2 which is localized in the cytoplasm²². Only the isoform 1 (with nuclear localization) is related to pluripotency. For example, McGuckin et al. report an Oct4 immunohistochemical staining to verify the pluripotent ES-like nature in CB cells⁶. Unfortunately, the OCT4 antibody used in their experiment additionally recognizes the OCT4 isoform 2, which has a cytoplasmic localization.
In order to evaluate the putative embryonic-like nature of CB derived cells USSCs, MNCs and CD34⁺ cells were analysed.
The expression of the pluripotency markers OCT4, NANOG, SOX2 has been studied as well as the transcription factors KLF4 and c-MYC utilized for the induction of pluripotent stem cells from adult human fibroblasts. Again, expression of these genes in any USSC cell lines, CD34⁺ cells and MNCs from CB tested (Fig.1A) has not been detected. Moreover, no evidence could be found for the expression in MSCs from bone marrow. As a positive control the embryonic carcinoma cell line NTERA-2 has been used showing a high expression of all three stem cell markers (see also Fig.1). The expression level of the transcription factors KLF4 and c-MYC was nearly equal in all USSC cell lines, clones from CB and BM MSCs. In the case of CB MNCs and CD34⁺ cells the expression was weak compared to the USSC and BM MSC samples. OCT4 real time PCRs has been performed to increase the sensitivity of the employed methods/ detection level. Nevertheless, USSCs, USSC clones, MNCs and CD34⁺ from CB and MSCs from bone marrow seem to reach OCT4 expression level comparable to human dermal fibroblasts (Fig.1B). To further confirm the RT-PCR results on protein level the expression of the stem cell markers OCT4 and NANOG was evaluated by immunohistochemistry. No OCT4 and NANOG expression in USSC cell lines, CD34⁺ cells (Fig.2), and MNCs (data not shown) from CB could be detected, in accordance with the other tumours and normal tissues investigated. Thus, it was concluded that neither cord blood subpopulations nor BM MSCs express the typical stem cell markers OCT4, NANOG and SOX2.
Further it was evaluated the expression of SSEA1, 3 and 4 by FACS analysis. The SSEA3 and SSEA4 antibodies respond to overlapping carbohydrate epitopes on globo-series gangliosids. During differentiation of ES and human embryonal carcinoma cells, the globo-series glygolipids defined by these antibodies decrease and the SSEA-1 antibody detectable lacto-series glycolipids appear. Human ESCs typically express SSEA3 and SSEA4 but not SSEA1, while their differentiation is characterized by the down regulation of SSEA3 and SSEA4 and up regulation of SSEA1. Human ES cells retain the earlier globo-series pathway and are SSEA3/4⁺ SSEA1⁻, whereas mouse ES cells use the lacto-series pathway and are thus SSEA3/4⁻ SSEA1⁺.

FACS analysis shows that neither USSC nor fresh isolated MNC from CB or BM express SSEA3, SSEA4 or SSEA1. Furthermore, it has been shown that the granulocytes from CB are SSEA1 positive.
One of the hallmarks of embryonic stem cells is the high expression of telomerase. The expression of telomerase permits embryonic stem cells to escape senescence.

Human telomeres save chromosomes from end-to-end fusion, recombination and degradation. Telomeres can be maintained by an enzyme called telomerase that adds 5'-TTAGGG-3' repeats to the ends of human chromosomes. Telomerase is composed of an RNA subunit (hTR) working as a template for telomeric repeats, a catalytic subunit (hTERT), and a telomerase associated protein and is proposed to be important for cell immortalization and cancer development. Serakinci et al. investigated the neoplastic potential of adult stem cells using MSCs transduced with a telomerase hTERT gene. It has been proved that telomerase-modified human MSC can extensively accumulate premalignant changes on continued division in culture. In some cases this leads to developing tumors in immunodeficient mice.

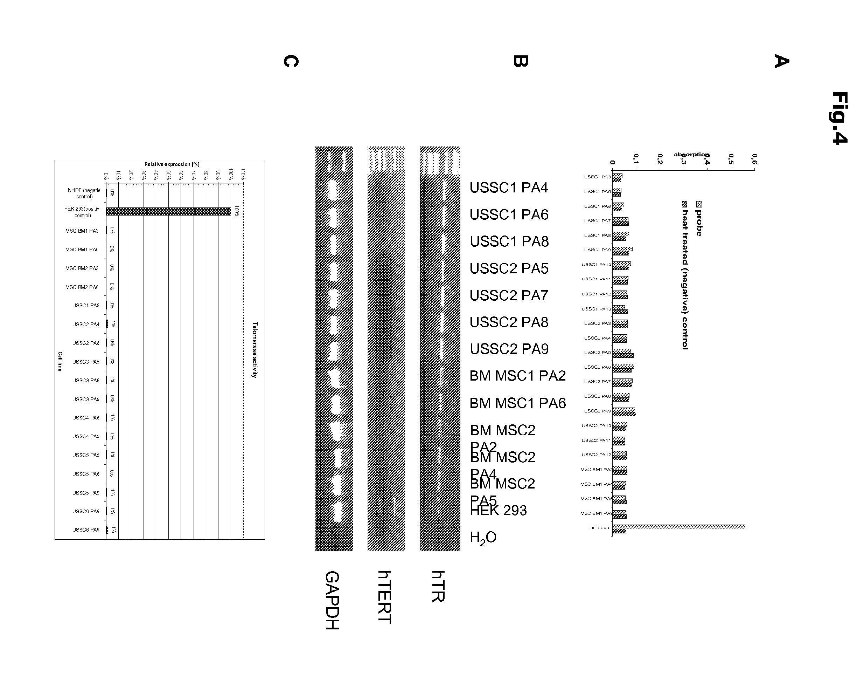

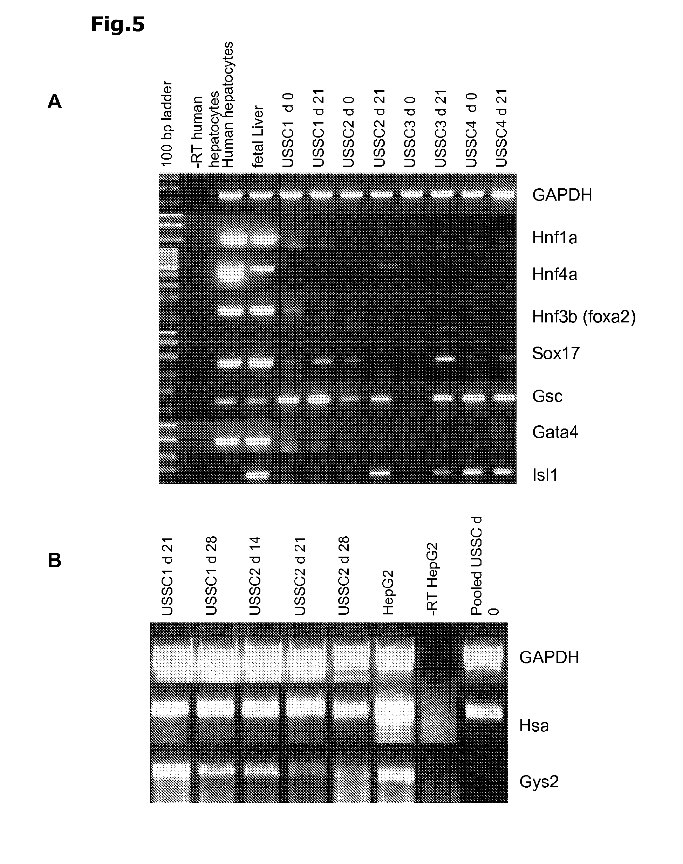

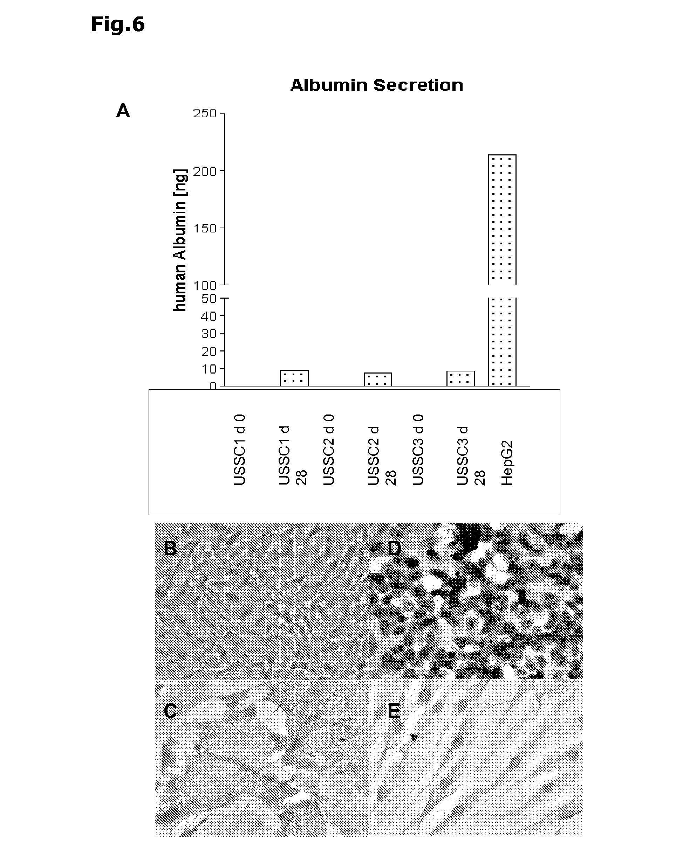

Different methods for detecting telomerase activity did not reveal any telomerase activity in cord blood cells or bone marrow.
The telomeric repeat amplification protocol (TRAP) assay revealed no telomerase activity in any passage, neither in USSC lines nor in MSC KM (Fig.4). The manufacturer's protocol suggests an at least two- fold stronger signal in probes compared to the control sample (heat treated probe). This had only been the case in the positive control HEK293, an immortal cell line expressing telomerase activity. As expected, the RT-PCR for hTR showed constitutive expressed telomerase RNA (Fig.4) in all cell lines and passages whereas hTERT, the catalytic subunit required for telomerase activity, is only expressed in immortal HEK293 cells. RT-PCR for hTERT was performed with primers capturing full-length transcript. A subsequent more sensitive real time experiment confirmed the data obtained according to the invention (Fig. 4).
In summary, the results of the invention indicate that USSCs and other subpopulations from cord blood are neonatal cells lacking the expression of typical embryonic stem cell markers like OCT4, NANOG and the transcription factor SOX2 and telomerase. It was shown already that fetal and neonatal stem cells could differ phenotypically and functionally from embryonic and from adult stem cells. For example, in hematopoietic stem cells (HSC) it was convincingly demonstrated that the transcription factor Sox17 is required for the maintenance of fetal and neonatal HSC and distinguishes their transcriptional regulation from adult HSCs.
Sox 17 has also been used as a marker of endodermal identity²³ and is required for the formation and maintenance of endoderm ^{24,25}. The invention has shown that beyond CB-USSC's ability to differentiate into mesodermal and ectodermal cells they are capable of generating an endodermal precursor phenotype². *In vitro* differentiation of USSCs triggered by growth factors, retinoic acid, matrigel matrix and oncostatin M induction led to changing their gene expression into a pattern which is typical for the endodermal linage. An increased expression of Sox17 and GSC as well as hsa, gys2 and HNF4 was detected by RT-PCR (Fig. 5). In addition, functional albumin secretion was observed (Fig. 6). Using PAS staining, glycogen storage in differentiated USSCs was indicated (Fig. 6 B-E).

Apparently, the ability of CB to differentiate into cells of all three germ layers does not require an embryonic-like phenotype, but further functional data have to show that CB can be compared to the adult counterpart, ES-cells and reprogrammed cells.

### Methods

### Isolation of primary cells and cell culture

### Total bone marrow and BM MNCs were obtained as descibed elsewere²⁶.

For assessment of total nucleated cells (TNC) from cord blood, aliquots of 3 ml were lysed with ammonium-chloride for 7 minutes and washed twice with PBS. For isolation of MNC from cord blood, a ficoll gradient (Biochrom, Berlin, Germany, density 1.077 g/cm³) followed by lysis of remaining red blood cells with ammonium chloride was applied. CD34⁺-cells were isolated out the MNC-fraction by using the magnetic activated cell sorting (MACS) isolation system (Milteny Biotec, Bergisch Gladbach, Germany) according to the instructions of the manufacturer, resulting in a purity of app. 95%. USSCs from cord blood and MSCs from bone marrow were isolated in accordance with informed donor consent and cultured as described elsewhere³. The mononuclear cells from cord blood were plated out at 5-7 x 10⁶ cells/ml in T25 culture flasks (Costar)containing DMEM, 30 % FCS (HyClone), dexamethasone (10⁻⁷ M; Sigma-Aldrich), penicillin (100 U/ml; Grünenthal), streptomycin (0.1 mg/ml; Hefa-pharma), and ultraglutamine (2 mM; Cambrex) Single cells were picked with the Aviso-cell selection system into 96 well plates. Expansion of the cells was performed in the same media in the absence of dexamethasone. Cells were incubated at 37°C in 5% CO₂ in a humidified atmosphere. When cells reached 80% confluency, they were detached with 0.25% trypsin (Cambrex)^{3,4,14}

### TRAP assay

Telomeric repeat amplification protocol (TRAP) assay was performed with a TRAP assay kit (Chemicon) according to the instructions of the manufacturer. RT-PCR, real time PCR and Taqman Assay

1*10⁶ cells were used for RNA-isolation with the RNeasy MiniKit (Qiagen) and eluted in 40µl DEPC dH₂O. 1µg of total RNA was reverse-transcribed into cDNA using the SuperScript® III First Strand Synthesis Kit (Invitrogen). The first strand synthesis was following the manufacturers' protocol. For the following PCR reaction 1 µl of cDNA was used as the template in a 50 µl final reaction volume comprising 1x PCR RXN buffer, 0.1 µM of each primer, 1.5mM MgCl₂, 0.2mM each dNTP and 1.25U *Taq* DNA Polymerase. PCR was performed in an Eppendorf Mastercycler EP.
Real time PCRs was performed using cDNA with SYBR Green PCR master mix from Applied Biosystems. Reactions were carried out in triplicates using ABI prism 7700.
Taq man Assay additionally revealed expression of Oct4. Assay was performed using Oct4 Taq Man Gene Expression Assay (Assay ID: Hs03005111g_1) and for reference reactions Taq Man Human GAPDH Endogenous Control. Taq Man PCR was performed on ABI PRISM 7700 Sequence Detection System (Applied Biosystems). Oct4 Assay, Master Mix and GAPDH Taq Man Probes were supplied from Applied Biosystems.

| **Primers** | **5' Sequence 3'** |
|---|---|
| **hTERT_F** | **GCC TGA GCT GTA CTT TGT CAA** |
| **hTERT_R** | **CGC AAA CAG CTT GTT CTC CA** |
| **GAPDH_F1** | **TTA GCA CCC CTG GCC AAG G** |
| **GAPDH_R1** | **CTT ACT CCT TGG AGG CCA TG** |
| **GAPDH_F2** | **GAG TCA ACG GAT TTG GTC GT** |
| **GAPDH_R2** | **TTG ATT TTG GAG GGA TCT CG** |
| **Oct4_F** | **AGC CCT CAT TTC ACC AGG CC** |
| **Oct4_R** | **TTG ATT TTG GAG GGA TCT CG** |
| **Nanog_F** | **TGA GAT GCC TCA CAC GGA G** |
| **Nanog_R** | **TTG CTC CAG GTT GAA TTG TTC** |
| **Sox2_F** | **AGA ACC CCA AGA TGC ACA AC** |
| **Sox2_R** | **ATG TAG GTC TGC GAG CTG GT** |
| Klf4_F | AGA AGG ATC TCG GCC AAT TT |
| Klf4_R | TGC TTG ACG CAG TGT CTT CT |
| Foxa2 For | GAG CGG TGA AGA TGG AAG G |
| Foxa2 Rev | TGT ACG TGT TCA TGC CGT TC |
| Hnf4a For | TAC TCC TGC AGA TTT AGC CG |
| Hnf4a Rev | GTC ATT GCC TAG GAG CAG C |
| Hnf1a For | CAG AGC CAT GTG ACC CAG AG |
| Hnf1a Rev | TGA GGT GAA GAC CTG CTT GG |
| Sox17 For | CAG AAT CCA GAC CTG CAC AA |
| Sox17 Rev | GCG GCC GGT ACT TGT AGT T |
| Gata4 For | TCC AAA CCA GAA AAC GGA AG |
| Gata4 Rev | TGC CCG TAG TGA GAT GAC AG |
| Gsc For | CAC CAT CTT CAC TGA CGA G |
| Gsc Rev | ACC AGA CCT CCA CTT TCT C |
| Isl1 For | TGA TGA AGC AAC TCC AGC AG |
| Isl1 Rev | TTT CCA AGG TGG CTG GTA AC |

### Table 1: Primer sequences used for RT-PCR analysis

Immunohistochemistry.
Cells were fixed with acetone and 4 % PFA respectively, washed with PBS, blocked in 5% normal goat serum in PBST (T=Tween20) and incubated with primary antibodies at 4 °C over night. For immunostaining following antibodies were used: Nanog (R&D Systems MAB1997) diluted 1:500, Oct4 (Santa Cruz sc5279) diluted 1:200. After washing, cells were incubated with secondary antibody, rhodamine Red^{™}-X-conjugated goat anti-mouse IgG (Dianova) for 1h, before being mounted in ProLong Gold antifade reagent with DAPI (Invitrogen). FACS Analysis

Flow cytometric analysis with unlabeled monoclonal antibodies against SSEA1 (clone MC480), SSEA3 (clone MC-631) and SSEA4 (clone MC-813-70) was performed as extra- as well as intracellular staining (all antibodies are from R&D systems, Minneapolis, MN; USA): as secondary antibody FITC labeled goat-anti-mouse IgG/IgM f(ab') fragment (Immunotech/ Beckman Coulter, Marseille, France) or Rhodamine Red^{™ -X} -labeled goat-anti-rat IgG +IgM(dianova, USA) was used. Intracellular staining was performed using the Cytofix/Cytoperm kit by Beckton Dickinson, Franklin Lakes, NJ, USA. Controls were assessed using the corresponding isotype (mouse IgM for SSEA1, rat IgM for SSEA3 and mouse IgG3 for SSEA4).

Endodermal Differentiation.
For endodermal differentiation USSCs, cloned single cells were plated at 1*10³ c/ cm² on 5 µg/ ml fibronectin coated 6-well plates (BD Bioscience) in DMEM (Lonza) supplemented with 30 % fetal calf serum (Cambrex/ Lonza). After 12-16 h cells were washed twice with PBS and cultured in IMDM (Gibco) supplemented with 5% fetal calf serum, 25 ng/ ml FGF4 and 25 ng/ BMP2 for 6 days. For additional 4 weeks, cells were cultured in serum free IMDM supplemented with 1X ITS (5 µg/ ml insulin, 5 µg/ ml transferrin, 5 ng/ ml selenium), 5*10⁻⁷ M dexamethason, 40 ng/ ml HGF (first two weeks) and 40 ng/ ml OSM (last two weeks) respectively. After 14, 21 and 28 days of HGF/ OSM induction cells were analyzed by RT-PCR, PAS and human albumin content in supernatant. All cytokines and growth factors were supplied by R&D Systems. ITS and dexamethason were supplied from Sigma-Aldrich.
Periodic Acid-Schiff Assay and Human Albumin ELISA.
PAS staining and measurement of human albumin content in supernatants by ELISA were performed as described previously ².

### Comparison of the cells of the invention and other cells of prior art

OBJECTIVES: Unrestricted somatic stem cells are non-haematopoietic stem cells from human umbilical cord blood. These cells, termed USSC, have been generated and characterized by our group since 2004 (Kögler et 81. 2004, Kögler et al. 2005, Kögler et al. 2006, Sensken et al. 2007, Liedtke et al. 2007). Even though USSC share many overlapping features with MSC from fetal tissue and bone marrow, such as the immunophenotype, osteogenic and chondrogenic differentiation potential in vitro as well as in vivo, USSC are a more immature precursor cell population.

MATERIALS: 25 USSC cell lineages and 20 cell clones isolated from a single cell with a AVISO cell selector from human umbilical cord blood and 3 MSC cell lineages from human bone marrow were differentiated into adipocytes.

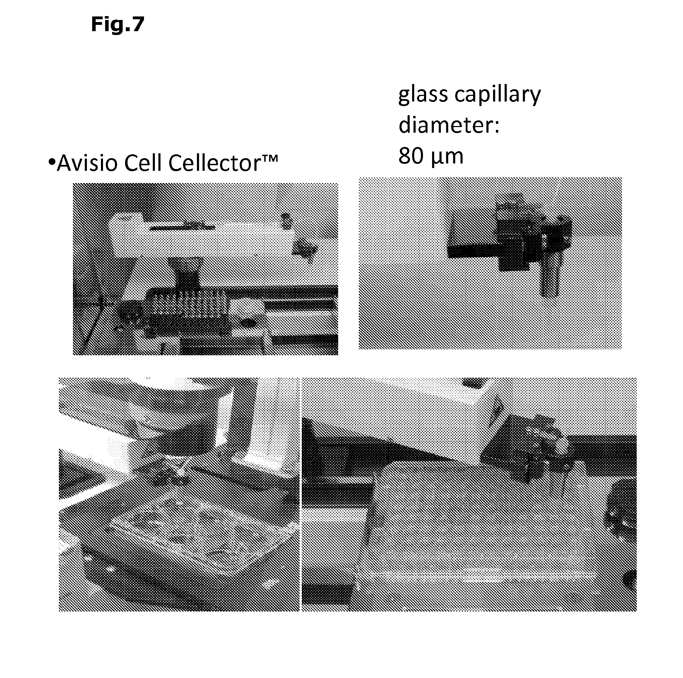

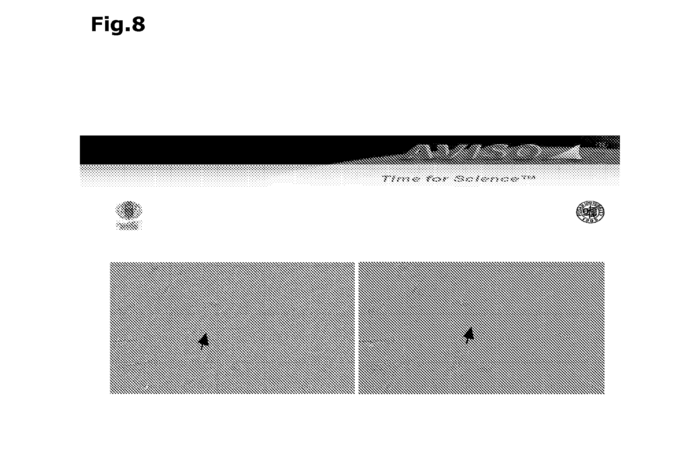

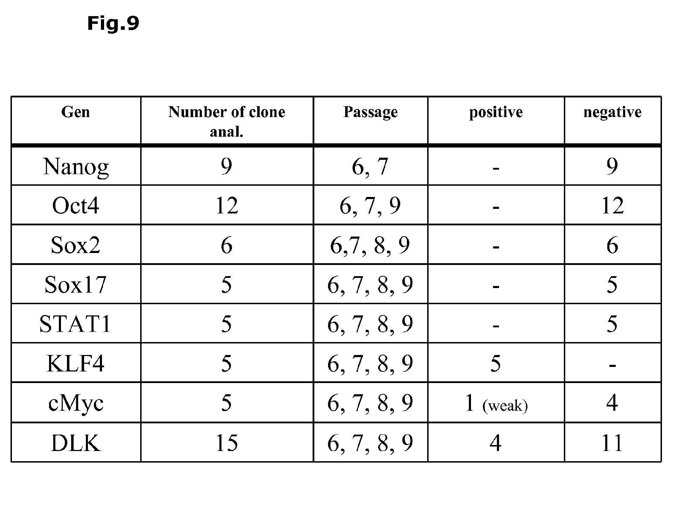

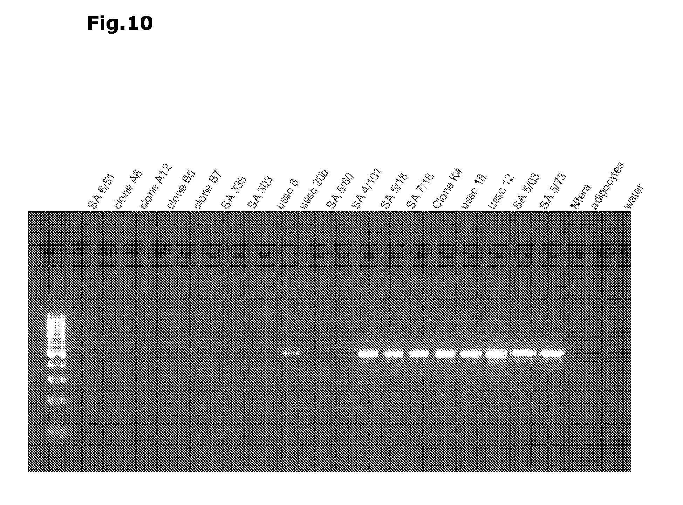

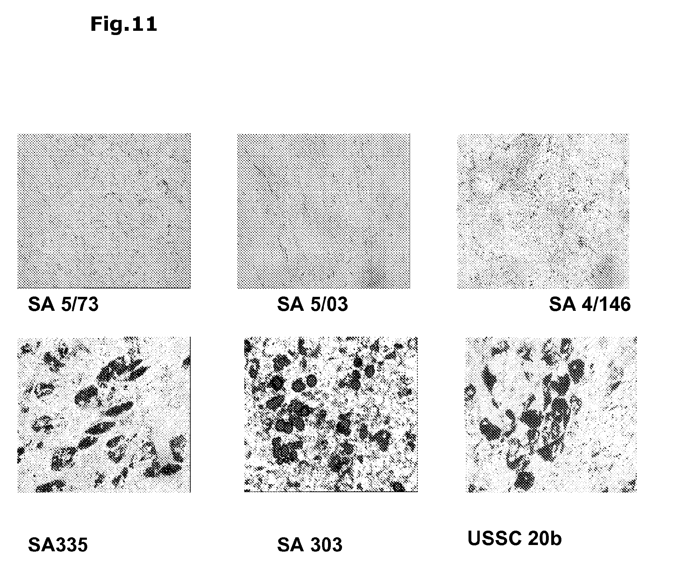

RESULTS (Figure 7-11): 14 out of 25 USSC cell lineages and all MSC cell lineages were differentiated into adipocytes after 14 days of adipogenic induction. None of these cell lineages expressed DLK1, correlated with an impaired differentiation-potential and reduced proliferative capacity. In 2 USSC cell lineages DLK1 could be detected only in passage 5, adipogenic differentiation was observed in passage 10. Cell clones from high DLK1 expressing USSC cell lineages adjusted DLK1 expression with increasing age. DLK1 expressing USSCs could not be differentiated into adipocytes in vitro, but a high neural and endodermal differentiation potential could be observed. Overexpression of DLK1 in weak expressing USSC Cell lineages resulted in higher differentiation potential of these cells, whereas adipogenic differentiation potential was dramatically reduced.

These findings suggest that there is a hierarchy of non-haematopoietic stem cells in cord blood which needs to be further clarified. Adipogenic differentiation potential as well as DLK1 expression of stem cells from cord blood allow a classification of these cells into MSC like stem cells, with low differentiation potential and reduced proliferation capacity beyond passage 6/7, and into pluripotent USSC which can be differentiated into cells of all three germ layers and therefore are a more immature cell population available on a clonal level.

References
1. Greschat, S. et al. Unrestricted somatic stem cells from human umbilical cord blood can be differentiated into neurons with a dopaminergic phenotype. Stem Cells Dev 17, 221-32 (2008).
2. Sensken, S. et al. In vitro differentiation of human cord blood-derived unrestricted somatic stem cells towards an endodermal pathway. Cytotherapy 9, 362-78 (2007).
3. Kogler, G. et al. A new human somatic stem cell from placental cord blood with intrinsic pluripotent differentiation potential. J Exp Med 200, 123-35 (2004).
4. Kogler, G., Sensken, S. & Wernet, P. Comparative generation and characterization of pluripotent unrestricted somatic stem cells with mesenchymal stem cells from human cord blood. Exp Hematol 34, 1589-95 (2006).
5. Baal, N. et al. Expression of transcription factor Oct-4 and other embryonic genes in CD133 positive cells from human umbilical cord blood. Thromb Haemost 92, 767-75 (2004).
6. McGuckin, C., Jurga, M., Ali, H., Strbad, M. & Forraz, N. Culture of embryonic-like stem cells from human umbilical cord blood and onward differentiation to neural cells in vitro. Nat Protoc 3, 1046-55 (2008).
7. McGuckin, C. et al. Embryonic-like stem cells from umbilical cord blood and potential for neural modeling. Acta Neurobiol Exp (Wars) 66, 321-9 (2006).
8. McGuckin, C. P. & Forraz, N. Potential for access to embryonic-like cells from human umbilical cord blood. Cell Prolif 41 Suppl 1, 31-40 (2008).
9. McGuckin, C. P. et al. Production of stem cells with embryonic characteristics from human umbilical cord blood. Cell Prolif 38, 245-55 (2005).
10. Habich, A. et al. Early appearance of stem/progenitor cells with neural-like characteristics in human cord blood mononuclear fraction cultured in vitro. Exp Hematol 34, 914-25 (2006).
11. Gang, E. J., Bosnakovski, D., Figueiredo, C. A., Visser, J. W. & Perlingeiro, R. C. SSEA-4 identifies mesenchymal stem cells from bone marrow. Blood 109, 1743-51 (2007).
12. Andrews, P. W. et al. Comparative analysis of cell surface antigens expressed by cell lines derived from human germ cell tumours. Int J Cancer 66, 806-16 (1996).
13. Jager, M. et al. [In vivo and in vitro bone regeneration from cord blood derived mesenchymal stem cells]. Orthopade 33, 1361-72 (2004).
14. Kogler, G. et al. Cytokine production and hematopoiesis supporting activity of cord blood-derived unrestricted somatic stem cells. Exp Hematol 33, 573-83 (2005).
15. Fallahi-Sichani, M. et al. In vitro differentiation of cord blood unrestricted somatic stem cells expressing dopamine-associated genes into neuron-like cells. Cell Biol Int 31, 299-303 (2007).
16. Kucia, M. et al. Morphological and molecular characterization of novel population of CXCR4+ SSEA-4+ Oct-4+ very small embryonic-like cells purified from human cord blood: preliminary report. Leukemia 21, 297-303 (2007).
17. Liedtke, S., Stephan, M. & Kögler, G. Oct4 expression revisited: Potential pitfalls for data misinterpretation in stem cell research. Biological Chemistry 389, 845-850 (2008).
18. Liedtke, S., Enczmann, J., Waclawczyk, S., Wernet, P. & Kogler, G. Oct4 and its pseudogenes confuse stem cell research. Cell Stem Cell 1, 364-6 (2007).
19. Suo, G. et al. Oct4 pseudogenes are transcribed in cancers. Biochem Bio-phys Res Commun 337, 1047-51 (2005).
20. Kotoula, V., Papamichos, S. I. & Lambropoulos, A. F. Revisiting OCT4 expression in peripheral blood mononuclear cells. Stem Cells 26, 290-1 (2008).
21. de Jong, J. & Looijenga, L. H. Stem cell marker OCT3/4 in tumor biology and germ cell tumor diagnostics: history and future. Crit Rev Oncog 12, 171-203 (2006).
22. Cauffman, G., Liebaers, I., Van Steirteghem, A. & Van de Velde, H. POU5F1 isoforms show different expression patterns in human embryonic stem cells and preimplantation embryos. Stem Cells 24, 2685-91 (2006).
23. Yasunaga, M. et al. Induction and monitoring of definitive and visceral endoderm differentiation of mouse ES cells. Nat Biotechnol 23, 1542-50 (2005).
24. Kanai-Azuma, M. et al. Depletion of definitive gut endoderm in Sox17-null mutant mice. Development 129, 2367-79 (2002).
25. Hudson, C., Clements, D., Friday, R. V., Stott, D. & Woodland, H. R. Xsox17alpha and -beta mediate endoderm formation in Xenopus. Cell 91, 397-405 (1997).
26. Battula, V. L. et al. Human placenta and bone marrow derived MSC cultured in serum-free, b-FGF-containing medium express cell surface frizzled-9 and SSEA-4 and give rise to multilineage differentiation. Differentiation 75, 279-91 (2007).

## Claims

1. Unrestricted somatic stem cell (USSC) derived from human umbilical cord blood, placental blood and/or blood samples from new-borns, said somatic stem cell being distinct from but capable to differentiate into mesenchymal cells (bone cells, chondrocytes), neural cells, endodermal cells, but not into haematopoietic lineage stem or progenitor cells and endothelial stem or progenitor cells
**characterized in that**
the USSC expresses the DLK1-gene the expression being detectable by a RT-PCR assay and/or on the protein level.

2. Stem cell of claim 1 adherent and CD45-, CD14-, CD34-negative.

3. Stem cell of claim 1 and/or 2 reacting negatively with the markers Oct4-, Nanog-, Sox 2, SSEA1, SSEA 3, and SSEA 4 and negative for telomerase activity

4. Medicaments comprising somatic stem cells of any of the claims 1 to 3.

5. Method of using unrestricted somatic stem cells according to any one of the claims 1 to 3 in gene therapy, organ regenartion or replacement, testing of pharmaceuticals, testing of biomaterials including orthopedic devices, in vitro and in vivo support and/or growth of blood vessels, in vitro and in vivo support of hematopoiesis, therapy of vascular and cardiac or smooth muscle diseases, bone, hepatic, pancreatic or neural diseases.

6. Method for the isolation and purification of unrestricted neonatal stem cells according to any of the claims 1 to 3 comprising the steps of density gradient isolation, selection and culture of adherent cells on a clonal level and subculture applying growth factors.
